# EUROPÄISCHE PATENTSCHRIFT

(11) **EP 2 392 360 B1**
(45) Veröffentlichungstag und Bekanntmachung des Hinweises auf die Patenterteilung: **20.09.2017**
(21) Anmeldenummer: 11003768.6
(22) Anmeldetag: 09.05.2011
(51) Int. Cl.: A61L 27/28, A61L 27/54

(54) **Antibiotische Beschichtung**
Antibiotic coating
Revêtement antibiotique

(30) Priorität: 19.05.2010 DE 102010020940
(43) Veröffentlichungstag der Anmeldung: 07.12.2011
(73) Patentinhaber: Heraeus Medical GmbH, 61273 Wehrheim (DE)
(72) Erfinder: Vogt, Sebastian, 99092 Erfurt (DE); Büchner, Hubert, 90491 Nürnberg (DE); Kühn, Klaus-Dieter, 35041 Marburg-Elnhausen (DE)
(74) Vertreter: Heraeus IP

(56) Entgegenhaltungen:
- EP-A2- 1 688 153
- US-A- 3 904 755
- US-A- 4 233 287
- US-A- 4 950 475
- US-A1- 2004 137 065

## Beschreibung

Die vorliegende Erfindung betrifft ein Implantat, das eine Beschichtung aufweist und ein Verfahren zur Herstellung eines solchen Implantats.

Die Implantation von Gelenkendoprothesen und auch von Osteosynthesematerialien ist immer mit der Gefahr einer mikrobiellen Kontamination verbunden. Wenn es mikrobiellen Keimen gelingt, sich an der Implantatoberfläche anzusiedeln, kann es zur Ausbildung einer postoperativen Osteitis kommen. Die Osteitis stellt eine schwerwiegende Komplikation für den Patienten dar. Es ist daher das Ziel, Implantate vor einer bakteriellen Besiedelung innerhalb der ersten Stunden bis Tage nach der Implantation zu schützen.

Seit Jahrzehnten wird mit klinischem Erfolg bei zementierten Gelenkendoprothesen mit Antibiotika dotierter PMMA-Knochenzement verwendet. Diese Entwicklung geht auf Buchholz und Engelbrecht zurück (H. W. Buchholz und E. Engelbrecht: Über die Depotwirkung einiger Antibiotika beim Vermischen mit dem Kunstharz Palacos. Chirurg. 41, 511-515, 1970). Dabei wird üblicherweise das Breitbandantibiotikum Gentamicin eingesetzt, das die Oberfläche des Knochenzementes wirksam gegen bakterielle Infektionen schützt.

Bei nicht-zementierten Gelenkendoprothesen und bei Osteosynthesematerialien wurde eine Reihe von Lösungswegen vorgeschlagen, um ebenfalls einen lokalen antibiotischen Schutz der Implantatoberflächen zu erreichen.

Diesen Lösungswegen liegt das Problem zugrunde, dass Antibiotika oder herkömmliche Antibiotikasalze nicht oder kaum auf der Oberfläche von Implantaten haften.

Daher wurde in einigen Patentschriften die Beschichtung von Implantaten mit bestimmten, in Wasser gering löslichen Antibiotikasalzen als antibiotisch wirkende Komponente vorgeschlagen. Exemplarisch sind hier EP 0 623 349 A1, EP 1 470 829 A1, EP 1 374 923 A2, DE 101 42 465 A1 und DE 44 04 018 A1 zu nennen. Diese in Wasser gering löslichen Salze lösen sich mit der Zeit unter Freisetzung der darin enthaltenen Antibiotika durch Einwirkung von Körperflüssigkeiten auf. Nachteilig dabei ist jedoch, dass sich diese Antibiotikasalze nur aufwendig herstellen lassen.

Aus dem Erfordernis, die Antibiotika in einer schwerlöslichen Form einzusetzen oder in eine solche zu überführen werden, um eine stabile Beschichtung zu erreichen, ergibt sich eine retardierte Freisetzung der Antibiotika. Wünschenswert ist aber oft gerade eine sofortige hohe und wirksame Antibiotika-Konzentration am Implantationsort.

Alternativ dazu ist es auch möglich, für Beschichtungen wasserlösliche Antibiotikasalze zu verwenden (V. Alt, A. Bitschnau, J. Osterling, A. Sewing, C. Meyer, R. Kraus, S. A. Meissner, S. Wenisch, E. Domann, R. Schnettler: The effects of combined gentamicin-hydroxylapatite coating for cementless joint prosthess on the reduction of infection rates in a rabbit infection prophylaxis model. Biomaterials 27 (26), 4627-34, 2006). Als Problem hat sich dabei aber erwiesen, dass sich die Antibiotika nur schwer auf der Implantatoberfläche fixieren lassen. Von Alt et al. wurde deshalb Gentamicin in eine poröse Hydroxylapatit-Beschichtung eingebracht, die als Trägermaterial dient. Hierbei ergibt sich jedoch das Problem, dass beim Abbau der Beschichtung im Körper toxische Produkte entstehen.

In US 2004/0137065 A1 wird eine Beschichtung beschrieben, welche zur Beschichtung von Medizinprodukten, die Kunststoffe enthalten, verwendet wird. Die Beschichtung enthält Antibiotika, welche in homogenen Polymermischungen suspendiert sind.

EP 1 688 153 A2) beschreibt die Verwendung einer Kombination von Glycerin und einem Weichmacher, bei dem es sich um Glycerin oder Sorbitol etc. handeln kann.

Viele Antibiotika weisen ferner die Tendenz zur Kristallisation auf. Dies gilt insbesondere für Lincosamide, Amikacin und Tobramycin. Werden diese Antibiotika zur Beschichtung von Implantaten eingesetzt, so besteht die Gefahr, dass diese Antibiotika kristallisieren und umliegendes Gewebe schädigen.

Der Erfindung liegt daher die Aufgabe zugrunde, ein Implantat mit einer antibiotischen Beschichtung zu schaffen, die kostengünstig und einfach herzustellen ist, gut und stabil an der Oberfläche des Implantats haftet, vom Körper ohne die Bildung von toxischen Produkten abgebaut werden kann, eine Kristallisation der Antibiotika in der Beschichtung verhindert und sofort eine hohe, lokal wirksame Antibiotika-Konzentration am Implantationsort gewährleistet. Die Beschichtung soll zudem auch an Oberflächen haften, die keine poröse Hydroxylapatitbeschichtung enthalten.

Diese Aufgabe wird gelöst durch ein Implantat, das eine Beschichtung aufweist, die wenigstens ein wasserlösliches Antibiotikum, Wasser und wenigstens ein Feuchthaltemittel, das aus der Gruppe ausgewählt ist, die aus wasserlöslichen Polyolen, ausgewählt aus Glycerin, Sorbitol, Mannitol, Glucitol, Isomalt, Lactit, Xylit, Threit, Erythrit, Arabit, 1,2-Ethandiol, 1,2-Propandiol und Dianhydroglycitol, und Aminosäuren besteht, enthält, wobei das Gewichtsverhältnis von dem wenigstens einen Antibiotikum und dem wenigstens einen Feuchthaltemittel im Bereich von 1000 : 1 bis 1 : 1 liegt.

Das erfindungsgemäße Implantat kann hergestellt werden, indem man das zu beschichtende Implantat auf eine Temperatur von wenigstens 90°C erwärmt und eine Beschichtungslösung auf das erwärmte, zu beschichtende Implantat aufbringt. Die Beschichtungslösung enthält wenigstens ein wasserlösliches Antibiotikum, Wasser und wenigstens ein Feuchthaltemittel, das aus der Gruppe ausgewählt ist, die aus wasserlöslichen Polyolen, ausgewählt aus Glycerin, Sorbitol, Mannitol, Glucitol, Isomalt, Lactit, Xylit, Threit, Erythrit, Arabit, 1,2-Ethandiol, 1,2-Propandiol und Dianhydroglycitol, und Aminosäuren besteht, wobei das Gewichtsverhältnis von dem wenigstens einen Antibiotikum und dem wenigstens einen Feuchthaltemittel im Bereich von 1000 : 1 bis 1 : 1 liegt

Die Erfindung beruht auf der überraschenden Erkenntnis, dass Antibiotika sehr gut haftende, zähe, lederartige Schichten ausbilden können, wenn diese geringe Mengen an Feuchtigkeit enthalten. Durch hygroskopisch wirkende Feuchthaltemittel kann ein Restwassergehalt in solchen Schichten erhalten werden. Dadurch ist es möglich, dass diese antibiotikahaltigen Schichten hinreichend mechanisch stabil bleiben und nicht zerfallen.

Im Rahmen der Erfindung werden als Implantate Materialien und Vorrichtungen verstanden, die im Zuge eines chirurgischen Eingriffes mindestens teilweise im Körperinneren eingebracht werden. Diese Implantate können im Kontakt zum Knochen und anderen Elementen des Stütz- und Bewegungsapparates sowie auch in Kontakt mit Blut oder Bindegewebe stehen. Bei den Implantaten kann es sich beispielsweise um Gelenkendoprothesen oder Osteosynthesematerialien handeln.

Unter Beschichtung wird erfindungsgemäß eine Schicht verstanden, die wenigstens eine Oberfläche des Implantats zumindest teilweise bedeckt. Gemäß einer bevorzugten Ausführungsform wird wenigstens eine Oberfläche des Implantats vollständig bedeckt.

Die Beschichtung enthält wenigstens ein Antibiotikum. Dieses Antibiotikum ist ein wasserlösliches Antibiotikum. Unter wasserlöslichem Antibiotikum wird ein Antibiotikum verstanden, das bei einer Temperatur von 25°C eine Löslichkeit in Wasser von wenigstens 5 g/l, vorzugsweise wenigstens 7 g/l und noch mehr bevorzugt wenigstens 10 g/l aufweist.

Die Antibiotika können in jeder Form vorliegen, in der das Antibiotikum antibiotische Wirksamkeit entfaltet oder die die Freisetzung einer Verbindung mit antibiotischer Wirkung ermöglicht.

Unter den Begriff Antibiotika fallen erfindungsgemäß demnach auch Antibiotikasalze oder Antibiotikaester sowie die entsprechenden hydratisierten Formen der Antibiotika, Antibiotikasalze oder Antibiotikaester.

Gemäß einer bevorzugten Ausführungsform kann das Antibiotikum aus der Gruppe ausgewählt sein, die aus Aminoglycosid-Antibiotika, Polypeptid-Antibiotika, Glykopeptiden, β-Lactamen, Polyketiden, Chinolonen und Sulfonamiden besteht.

Bei den Aminoglycosid-Antibiotika kann es sich beispielsweise um Amikacyn, Apramycin, Geneticin, Gentamicin, Kanamycin, Netilmicin, Neomycin, Paromomycin, Spectinomycin, Streptomycin oder Tobramycin handeln. Gemäß einer besonders bevorzugten Ausführungsform handelt es sich bei dem Aminoglycosid-Antibiotikum um Gentamicin oder ein Gentamicin-Derivat. Als Gentamicin-Derivat kommen vorzugsweise Gentamicinsalze oder Gentamicinester in Betracht. Als beispielhaftes Gentamicinsalz sei Gentamicinsulfat genannt. Gentamicinsulfat ist ein in der Chirurgie und Orthopädie seit Jahrzehnten bewährtes, kostengünstiges Breitbandantibiotikum. Pharmakopoe konformes Gentamicinsulfat stellt ein Gemisch aus den Gentamicinhomologen C1a, C1, C2a, C2b und C2 dar. Aufgrund des Vorliegens als Gemisch mehrerer Gentamicinhomologen kristallisiert dieses Antibiotikum nicht. Gentamicin hat gegenüber anderen Antibiotika die herausragende Eigenschaft, dass es kurzzeitig höhere Temperaturen ohne Verlust der antimikrobiellen Wirksamkeit tolerieren kann.

Als Polypeptid-Antibiotika kommen unter anderem Polymyxine, Bacitracin und Tyrothricin in Betracht.

Als β-Lactame können erfindungsgemäß auch zum Beispiel Penicilline, Cephalosporine, Monobactame und Carbapeneme eingesetzt werden.

Als Polyketide können unter anderem Tetracycline oder Makrolid-Antibiotika, wie zum Beispiel Erythromycin, Verwendung finden.

Die Beschichtung enthält ferner ein Feuchthaltemittel. Dieses Feuchthaltemittel erfüllt die Aufgabe, die Beschichtung auf einen gewünschten Restfeuchtegehalt einzustellen. Erfindungsgemäß wird als Feuchthaltemittel eine Verbindung eingesetzt, die aus der Gruppe ausgewählt ist, die aus wasserlöslichen Polyolen und Aminosäuren besteht.

Die Polyole sind erfindungsgemäß aus der Gruppe ausgewählt, die aus Glycerin, Sorbitol, Mannitol, Glucitol, Isomalt, Lactit, Xylit, Threit, Erythrit, Arabit, 1,2-Ethandiol, 1,2-Propandiol und Dianhydroglycitol besteht.

Die Beschichtung des Implantats kann ferner eine Aminosäure aufweisen.

Bei der Aminosäure kann es sich um eine essentielle oder eine nicht-essentielle Aminosäure handeln.

Die Aminosäure kann eine aliphatische, aromatische oder heterozyklische Aminosäure sein, wobei jedoch aliphatische Aminosäuren bevorzugt sind.

Vorzugsweise weist die Aminosäure eine molare Masse im Bereich von 75 - 200 g/mol auf.

Gemäß einer besonders bevorzugten Ausführungsform handelt es sich bei der Aminosäure um Glycin.

Die erfindungsgemäßen Feuchthaltemittel bewirken, dass in der Beschichtung des Implantats eine Restfeuchtigkeit verbleibt und das Antibiotikum dadurch als sehr zähe, amorphe Schicht auf Substraten, vorzugsweise Metalloberflächen, haftet. Durch das Verbleiben einer Restfeuchte kann ferner eine Kristallisation der Antibiotika wirksam verhindert werden.

Der Anteil von Wasser an der Beschichtung, bezogen auf die Masse der Beschichtung des Implantats, liegt vorzugsweise im Bereich von 5 - 30 Gewichtsprozent und noch mehr bevorzugt im Bereich von 7 - 28 Gewichtsprozent.

Bei einer Implantation des erfindungsgemäßen Implantats löst sich die Beschichtung durch Einwirkung von Körperflüssigkeiten, wie Blut und Wundsekret, innerhalb von wenigen Minuten bis Stunden auf. Dadurch wird eine sofortige hohe und wirksame Antibiotika-Konzentration am Implantationsort sichergestellt. Das in der Beschichtung enthaltene Antibiotikum wird unmittelbar an der Grenzfläche zwischen dem Implantat und dem Knochen- bzw. Weichgewebe freigesetzt und schützt die Implantatoberfläche so wirksam vor einer bakteriellen Besiedlung.

Die Beschichtung des Implantats kann erfindungsgemäß weitere Bestandteile enthalten. Beispielsweise kann die Beschichtung wenigstens ein weiteres Antiseptikum oder Hämostyptikum aufweisen.

Unter dem Begriff Antiseptika sind alle in der Medizin üblichen antiseptischen Substanzen zu verstehen. Besonders bevorzugt sind dabei Octenidindihydrochlorid, Polyhexanid und quartäre Ammoniumverbindungen, wie zum Beispiel Benzalkoniumchlorid.

Unter dem Begriff Hämostyptikum sind Substanzen zu verstehen, die die Blutgerinnung aktivieren. Als Hämostyptika sind insbesondere Calciumsalze bevorzugt. Gemäß einer bevorzugten Ausführungsform ist das Calciumsalz aus der Gruppe ausgewählt, die aus Calciumchlorid, Calciumacetat und Calciumlactat besteht.

Erfindungsgemäß kann auf der Beschichtung des Implantats eine Deckschicht angeordnet sein. Diese Deckschicht enthält vorzugsweise biokompatible Filmbildner. Filmbildner sind Verbindungen, die in der Lage sind, auf einer Oberfläche eine Schicht auszubilden. Gemäß einer bevorzugten Ausführungsform können als Filmbildner filmbildende Antibiotikasalze, Antibiotikaester Antiseptikasalze und/oder Antiseptikaester eingesetzt werden. Daneben können jedoch auch weitere Filmbildner, insbesondere polymere Filmbildner, wie zum Beispiel Methylcellulose, Polyvidonacetat, Hydroxypropylmethylcellulosephthalat, Carboxymethylcellulose, Polyvinylacetatphthalat, Schellack oder Methacrylsäureester verwendet werden.

Die Beschichtung des erfindungsgemäßen Implantats enthält vorzugsweise 0,01 - 80 Gewichtsprozent des wenigstens einen Antibiotikums, 0,01 - 80 Gewichtsprozent des wenigstens einen Feuchthaltemittels, 5 - 30 Gewichtsprozent Wasser und 0 - 50 Gewichtsprozent weitere Bestandteile, bezogen auf das Gewicht der Beschichtung.

Erfindungsgemäß liegt das Gewichtsverhältnis von dem wenigstens einen Antibiotikum und dem wenigstens einen Feuchthaltemittel im Bereich von 1000 : 1 bis 1 : 1.

Bei der Herstellung des erfindungsgemäßen Implantats kann eine Beschichtungslösung zum Einsatz kommen. Diese Beschichtungslösung enthält wenigstens ein Antibiotikum, Wasser und wenigstens ein Feuchthaltemittel, das aus der Gruppe ausgewählt ist, die aus wasserlöslichen Polyolen und Aminosäuren besteht, wobei die Beschichtungslösung wenigstens ein wasserlösliches Antibiotikum, Wasser und wenigstens ein Feuchthaltemittel, das aus der Gruppe ausgewählt ist, die aus wasserlöslichen Polyolen ausgewählt aus Glycerin, Sorbitol, Mannitol, Glucitol, Isomalt, Lactit, Xylit, Threit, Erythrit, Arabit, 1,2-Ethandiol, 1,2-Propandiol und Dianhydroglycitol, und Aminosäuren besteht, enthält, wobei das Gewichtsverhältnis von dem wenigstens einen Antibiotikum und dem wenigstens einen Feuchthaltemittel im Bereich von 1000 : 1 bis 1 : 1 liegt.

Im Zusammenhang mit dem wenigstens einen Antibiotikum der Beschichtungslösung und dem wenigstens einen Feuchthaltemittel wird auf die vorstehenden Ausführungen Bezug genommen.

Die Beschichtungslösung enthält gemäß einer bevorzugten Ausführungsform 0,01 - 40 Gewichtsprozent des wenigstens einen Antibiotikums, 0,01 - 40 Gewichtsprozent des wenigstens einen Feuchthaltemittels, 10 - 99,5 Gewichtsprozent Wasser und 0 - 50 Gewichtsprozent weitere Bestandteile.

Das erfindungsgemäße Implantat kann auf verschiedene Weisen hergestellt werden.

Erfindungsgemäß kann das beschichtete Implantat hergestellt werden, indem man zunächst das zu beschichtende Implantat bereitstellt und auf eine Temperatur von wenigstens 90°C, vorzugsweise wenigstens 100°C, erwärmt.

Anschließend kann die erfindungsgemäße Beschichtungslösung auf das erwärmte, zu beschichtende Implantat aufgetragen werden.

Als vorteilhaft hat sich dabei erwiesen, die Beschichtungslösung unmittelbar vor dem Auftragen auf das Implantat auf eine Temperatur von mehr als 40°C, vorzugsweise mehr als 50°C, zu erwärmen. Dadurch verdampft das in der Beschichtungslösung enthaltene Wasser schnell beim Aufbringen auf das erwärmte Substrat.

Das Auftragen der Beschichtungslösung auf das Implantat kann beispielsweise durch Aufsprühen erfolgen. Beim Auftreffen des Sprühnebels auf das erwärmte Substrat verdampft das Wasser nahezu vollständig. Dabei verbleibt als Beschichtung auf dem Implantat das wenigstens eine Antibiotikum mit dem wenigstens einen Feuchthaltemittel, wobei die Gegenwart des wenigstens einen Feuchthaltemittels bedingt, dass ein Teil des Wassers aus der Beschichtungslösung in der Beschichtung zurückgehalten wird. Dadurch bildet sich eine hornartige Schicht auf dem Substrat aus.

Gegebenenfalls kann im Anschluss eine Nachtrocknung der Beschichtung erfolgen. Diese Nachtrocknung kann im Warmluftstrom oder im Vakuum durchgeführt werden. Es ist weiterhin auch möglich, die Beschichtung durch Einwirkung von Mikrowellenstrahlung zu trocknen. Ebenfalls ist es bei der Beschichtung von metallischen Implantaten möglich, durch Einwirkung von magnetischen Wechselfeldern eine induktive Erwärmung der Implantate vorzunehmen, um eine schnelle Trocknung der Beschichtung zu erreichen.

Soll das beschichtete Implantat mit einer Deckschicht versehen werden, so kann beispielsweise während der Trocknung der Beschichtung ein pulverförmiger Filmbildner, zum Beispiel pulverförmige Antibiotika und/oder Antiseptika, auf die noch klebende Beschichtung aufgebracht werden. Die Aufbringung des Filmbildners kann durch Besprühen oder auch durch Tauchen in ein Bad mit dem pulverförmigen Wirkstoff erfolgen.

Eine andere Möglichkeit, das beschichtete Implantat mit einer Deckschicht zu versehen, besteht darin, eine Lösung eines Filmbildners, beispielsweise eine alkoholische Lösung eines Antibiotikums, auf das beschichtete Implantat aufzutragen. Hierzu wird das beschichtete Implantat vorzugsweise zunächst auf eine Temperatur von wenigstens 90°C, mehr bevorzugt wenigstens 100°C, erwärmt und die Lösung des Filmbildners anschließend unter Verdampfung des Lösungsmittels auf das beschichtete Implantat aufgebracht.

### Beispiele:

Die Erfindung wird durch die nachstehenden Beispiele näher erläutert, ohne die Erfindung jedoch zu beschränken.

### Beispiel 1:

Es wurden 20 g Gentamicinsulfat (Hersteller Fujian Fukang Ltd. China) und 0,1 g Glycerin in 80 g Wasser gelöst. Es entstand eine klare, schwach gelbliche Lösung. Diese Lösung wurde mit Hilfe einer Druckluftsprühpistole auf eine zuvor auf 120 °C erwärmte Titanscheibe (Durchmesser 1,5 cm) gesprüht. Ein Teil des Wassers verdampfte und es bildete sich eine gleichmäßige, hornartige Schicht auf der Titanscheibe aus. Die beschichtete Titanscheibe wurde bei 100°C im Trockenschrank bis zur Massekonstanz getrocknet und die Masse der Beschichtung gravimetrisch bestimmt. Die Masse der Beschichtung betrug 1 mg.

### Beispiel 2:

Es wurden 20 g Gentamicinsulfat (Hersteller Fujian Fukang Ltd. China), 0,1 g Triethylenglykol (Fluka) und 0,05 g Calciumchlorid (Fluka) in 80 g Wasser gelöst. Es entstand eine klare, schwach gelbliche Lösung. Diese Lösung wurde mit Hilfe einer Druckluftsprühpistole auf eine zuvor auf 120 °C erwärmte Titanscheibe (Durchmesser 1,5 cm) gesprüht. Ein Teil des Wassers verdampfte und es bildete sich eine gleichmäßige, hornartige Schicht auf der Titanscheibe aus. Die beschichtete Titanscheibe wurde bei 100°C im Trockenschrank bis zur Massekonstanz getrocknet und die Masse der Beschichtung gravimetrisch bestimmt. Die Masse der Beschichtung betrug 0,9 mg.

### Beispiel 3:

Es wurden 20 g Gentamicinsulfat (Hersteller Fujian Fukang Ltd. China), 0,05 g Glycerin (Fluka) und 0,05 g Benzalkoniumchlorid in 80 g Wasser gelöst. Es entstand eine klare, schwach gelbliche Lösung. Diese Lösung wurde mit Hilfe einer Druckluftsprühpistole auf eine zuvor auf 120 °C erwärmte Titanscheibe (Durchmesser 1,5 cm) gesprüht. Ein Teil des Wassers verdampfte und es bildete sich eine gleichmäßige, hornartige Schicht auf der Titanscheibe aus. Die beschichtete Titanscheibe wurde bei 100°C im Trockenschrank bis zur Massekonstanz getrocknet und die Masse der Beschichtung gravimetrisch bestimmt. Die Masse der Beschichtung betrug 0,9 mg.

### Beispiel 4:

Es wurden 20 g Gentamicinsulfat (Hersteller Fujian Fukang Ltd. China), 0,05 g Glycerin (Fluka), 0,05 g Benzalkoniumchlorid und 0,05 g Calciumchlorid in 80 g Wasser gelöst. Diese Lösung wurde mit Hilfe einer Druckluftsprühpistole auf eine zuvor auf 120 °C erwärmte Titanscheibe (Durchmesser 1,5 cm) gesprüht. Ein Teil des Wassers verdampfte und es bildete sich eine gleichmäßige, hornartige Schicht auf der Titanscheibe aus. Die beschichtete Titanscheibe wurde bei 100°C im Trockenschrank bis zur Massekonstanz getrocknet und die Masse der Beschichtung gravimetrisch bestimmt. Die Masse der Beschichtung betrug 1,0 mg.

### Beispiel 5:

Es wurden 20 g Gentamicinsulfat (Hersteller Fujian Fukang Ltd. China) und 0,05 g Glycerin (Fluka) in 80 g Wasser gelöst. Es entstand eine klare, schwach gelbliche Lösung. Diese Lösung wurde mit Hilfe einer Druckluftsprühpistole auf eine zuvor auf 120 °C erwärmte Titanscheibe (Durchmesser 1,5 cm) gesprüht. Ein Teil des Wassers verdampfte und es bildete sich eine gleichmäßige, hornartige Schicht auf der Titanscheibe aus. Die beschichtete Titanscheibe wurde bei 100°C im Trockenschrank bis zur Massekonstanz getrocknet und die Masse der Beschichtung gravimetrisch bestimmt. Die Masse der Beschichtung betrug 0,9 mg. Danach wurde die getrocknete, beschichtete Titanscheibe auf 100 °C erwärmt und mit einer 4 %igen methanolischen Gentamicinpalmitat-Lösung besprüht. Dabei verdampfte das Methanol und es bildete sich eine Deckschicht aus Gentamicinpalmitat aus. Nach Trocknung bis zur Massekonstanz wurde die die so beschichtete Titanscheibe nochmals ausgewogen. Die Deckschicht hatte eine Masse von 1,3 mg.

## Patentansprüche

1. Implantat aufweisend eine Beschichtung, die wenigstens ein wasserlösliches Antibiotikum, Wasser und wenigstens ein Feuchthaltemittel, das aus der Gruppe ausgewählt ist, die aus wasserlöslichen Polyolen ausgewählt aus Glycerin, Sorbitol, Mannitol, Glucitol, Isomalt, Lactit, Xylit, Threit, Erythrit, Arabit, 1,2-Ethandiol, 1,2-Propandiol und Dianhydroglycitol, und Aminosäuren besteht, enthält, wobei das Gewichtsverhältnis von dem wenigstens einen Antibiotikum und dem wenigstens einen Feuchthaltemittel im Bereich von 1000 : 1 bis 1 : 1 liegt.

2. Implantat nach Anspruch 1, **dadurch gekennzeichnet, dass** das wasserlösliche Antibiotikum ein Gentamicinsalz ist.

3. Implantat nach Anspruch 2, **dadurch gekennzeichnet, dass** das Gentamicinsalz Gentamicinsulfat ist.

4. Implantat nach Anspruch 1, **dadurch gekennzeichnet, dass** die Aminosäure Glycin ist.

5. Implantat nach einem der Ansprüche 1 - 4, **dadurch gekennzeichnet, dass** die Beschichtung wenigstens einen weiteren Stoff enthält, der aus der Gruppe ausgewählt ist, die aus Antiseptika, Antiphlogistika und Hämostyptika besteht.

6. Verfahren zur Herstellung eines beschichteten Implantats nach einem der Ansprüche 1 - 5, bei dem man das zu beschichtende Implantat auf eine Temperatur von wenigstens 90°C erwärmt und eine Beschichtungslösung auf das erwärmte, zu beschichtende Implantat aufbringt, wobei die Beschichtungslösung wenigstens ein wasserlösliches Antibiotikum, Wasser und wenigstens ein Feuchthaltemittel, das aus der Gruppe ausgewählt ist, die aus wasserlöslichen Polyolen ausgewählt aus Glycerin, Sorbitol, Mannitol, Glucitol, Isomalt, Lactit, Xylit, Threit, Erythrit, Arabit, 1,2-Ethandiol, 1,2-Propandiol und Dianhydroglycitol, und Aminosäuren besteht, enthält, wobei das Gewichtsverhältnis von dem wenigstens einen Antibiotikum und dem wenigstens einen Feuchthaltemittel im Bereich von 1000 : 1 bis 1 : 1 liegt.

## Claims

1. Implant, comprising a coating that contains at least one water-soluble antibiotic, water, and at least one humectant selected from the group consisting of water-soluble polyols selected from glycerol, sorbitol, mannitol, glucitol, isomalt, lactitol, xylitol, threitol, erythritol, arabitol, 1,2-ethanediol, 1,2-propanediol, and dianhydroglycitol, and amino acids, whereby the weight ratio of the at least one antibiotic and the at least one humectant is in the range of 1000 : 1 to 1 : 1.

2. Implant according to claim 1, **characterised in that** the water-soluble antibiotic is a gentamicin salt.

3. Implant according to claim 2, **characterised in that** the gentamicin salt is gentamicin sulfate.

4. Implant according to claim 1, **characterised in that** the amino acid is glycine.

5. Implant according to any one of the claims 1 - 4, **characterised in that** the coating contains at least one further substance that is selected from the group consisting of antiseptics, and antiphlogistics, and hemostyptics.

6. Method for the production of a coated implant according to any one of the claims 1 - 5, in which the implant to be coated is heated to a temperature of at least 90°C and a coating solution is applied to the heated implant to be coated, whereby the coating solution contains at least one water-soluble antibiotic, water, and at least one humectant selected from the group consisting of water-soluble polyols selected from glycerol, sorbitol, mannitol, glucitol, isomalt, lactitol, xylitol, threitol, erythritol, arabitol, 1,2-ethanediol, 1,2-propanediol, and dianhydroglycitol, and amino acids, whereby the weight ratio of the at least one antibiotic and the at least one humectant is in the range of 1000 : 1 to 1 : 1.

## Revendications

1. Implant présentant un revêtement qui contient au moins un antibiotique hydrosoluble, de l'eau et au moins un humectant qui est sélectionné parmi le groupe qui se compose de polyols hydrosolubles sélectionnés parmi la glycérine, le sorbitol, le mannitol, le glucitol, l'isomalt, le lactitol, le xylitol, le thréitol, l'érythritol, l'arabitol, le 1,2-éthanediol, le 1,2-propanediol et le dianhydroglycitol, et d'acides aminés, dans lequel le rapport pondéral de l'au moins un antibiotique et l'au moins un humectant se situe dans la plage de 1000/1 à 1/1.

2. Implant selon la revendication 1, **caractérisé en ce que** l'antibiotique hydrosoluble est un sel de gentamicine.

3. Implant selon la revendication 2, **caractérisé en ce que** le sel de gentamicine est le sulfate de gentamicine.

4. Implant selon la revendication 1, **caractérisé en ce que** l'acide aminé est la glycine.

5. Implant selon une des revendications 1 à 4, **caractérisé en ce que** le revêtement contient au moins une autre substance qui est sélectionnée parmi le groupe qui se compose d'antiseptiques, d'anti-inflammatoires et d'antihémorragiques.

6. Procédé de fabrication d'un implant revêtu selon une des revendications 1 à 5, lors duquel on chauffe l'implant à revêtir à une température d'au moins 90°C et applique une solution de revêtement sur l'implant chauffé, à revêtir, dans lequel la solution de revêtement contient au moins un antibiotique hydrosoluble, de l'eau et au moins un humectant qui est sélectionné parmi le groupe qui se compose de polyols hydrosolubles sélectionnés parmi la glycérine, le sorbitol, le mannitol, le glucitol, l'isomalt, le lactitol, le xylitol, le thréitol, l'érythritol, l'arabitol, le 1,2-éthanediol, le 1,2-propanediol et le dianhydroglycitol, et d'acides aminés, dans lequel le rapport pondéral de l'au moins un antibiotique et l'au moins un humectant se situe dans la plage de 1000/1 à 1/1.
